# EUROPEAN PATENT SPECIFICATION

(11) **EP 3 137 141 B1**
(45) Date of publication and mention of the grant of the patent: **23.06.2021**
(21) Application number: 15786819.1
(22) Date of filing: 01.05.2015
(51) Int. Cl.: A61M 15/06

(54) **VAPORIZER**
VERDAMPFER
VAPORISATEUR

(30) Priority: 01.05.2014 US 201461987196 P; 18.08.2014 US 201462038785 P
(43) Date of publication of application: 08.03.2017
(73) Proprietor: VMR Products, LLC, Miami, FL 33137 (US)
(72) Inventor: VERLEUR, Jan, Andries, Miami, FL 33132 (US); RECIO, Dan, Miami, FL 33137 (US); LU, Yifeng, Miami, FL 33137 (US); FAJARDO, Arturo, Frisco, TX 75034 (US)
(74) Representative: Thum, Bernhard
(86) International application number: PCT/US2015/028909
(87) International publication number: WO 2015/168631

(56) References cited:
- EP-A1- 2 260 733
- CN-A- 103 349 363
- CN-A- 103 750 571
- CN-U- 202 566 289
- CN-U- 203 234 036
- US-A- 4 569 136
- US-A- 4 569 136
- US-A1- 2011 220 234
- US-A1- 2013 152 922
- US-A1- 2013 298 905
- US-A1- 2013 298 905
- US-A1- 2014 007 863

## Description

### CROSS-REFERENCE TO RELATED APPLICATIONS

This application claims priority to U.S. Provisional Application No. 61/987,196 filed on May 1, 2014, U.S. Provisional Application No. 62/038,785 filed on August 18, 2014, and U.S. Design Patent Application No. 29/498,556 filed on August 05, 2014.

### FIELD OF THE DISCLOSURE

This disclosure relates generally to vaporizers, which may also be referred to as electronic cigarettes.

### BACKGROUND

Vaporizers have recently emerged as a new product for providing nicotine through a smokeless inhalation process. There are many embodiments of the electronic cigarette. Most implementations consist of a power supply (typically a battery) and an atomizing device. In reusable electronic cigarettes the two items are separated into a battery and a cartomizer, to allow the disposal and replacement of the nicotine containing fluid cartomizer while preserving the more costly battery and associated circuitry (microcontroller, switch, indicating LED, etc.) In disposable electronic cigarettes the two items are combined to integrate the functions into one unit that is disposed of after either the battery energy or the nicotine containing liquid is exhausted.

The liquid that is used to produce vapor in electronic cigarettes is generally a solution of one or more of propylene glycol (PG) and/or vegetable glycerin (VG) and/or polyethylene glycol 400 (PEG400) mixed with concentrated flavors, and optionally, a variable percentage of a liquid nicotine concentrate. The solution is often sold in a bottle or in disposable cartridges or cartomizers. Many different flavors of such liquid are sold, including flavors that resemble the taste of regular tobacco, menthol, vanilla, coffee, cola and various fruits. Various nicotine concentrations are also available, and nicotine-free solutions are also common.

Documents CN 202 566 289 U and US 2014/007863 A1 disclose vaporizer according to the preamble of claim 1.

### BRIEF SUMMARY OF THE DISCLOSURE

The following presents a simplified summary of the disclosure in order to provide a basic understanding of some aspects of the invention. This summary is not an extensive overview of every embodiment disclosed herein. It is intended to neither identify key or critical elements of the various embodiments nor delineate the scope of the disclosure. Its sole purpose is to present some concepts of the disclosure, in accordance with the various embodiments disclosed herein, in a simplified form as a prelude to the more detailed description that is presented later.

In one embodiment of the disclosure, a vaporizer may include a cartomizer and a battery segment. The cartomizer have may have a first cartomizer end and a second cartomizer end. The cartomizer may include an outer cartomizer shell, an internal cavity defined by a portion of the outer cartomizer shell, a heating chamber provided within the outer cartomizer shell and adjacent to the internal cavity, a heating element provided within the heating chamber, a fluid container conformingly dimensioned with the internal cavity, the fluid container insertable and removable from the internal cavity, a mouthpiece provided at or proximate to the second cartomizer end, an airflow channel in fluid communication with the heating chamber and the mouthpiece, cartomizer electrical contacts provided on the outer cartomizer shell proximate the first cartomizer end, and cartomizer electrical circuitry operable to direct an electric current between the cartomizer electrical contacts and the heating element. The battery segment may include an outer battery shell having a first battery end and a second battery end, the cartomizer connectable with the battery segment proximate the first cartomizer end and the first battery end, a battery housed within the outer battery shell, battery electrical contacts provided proximate the first battery end and positioned to contact the cartomizer electrical contacts when the cartomizer is connected to the battery segment, and battery electrical circuitry housed within the battery segment and operable to direct an electrical current between the battery, the battery electrical contacts, the cartomizer electrical contacts, and the heating element. The battery segment further includes a printed circuit board connected to the battery electrical circuitry, wherein the printed circuit board extends through at least a portion of the outer battery shell between the battery and the longitudinal wall of the outer battery shell.

The following description and the annexed drawings set forth certain illustrative aspects of the embodiments of the disclosure. These aspects are indicative, however, of but a few of the various ways in which the principles of the disclosure may be employed and the various embodiments are intended to include all such aspects and their equivalents. Other advantages and novel features will become apparent from the following description when considered in conjunction with the drawings.

### BRIEF DESCRIPTION OF THE FIGURES

FIG. 1 illustrates side view of an exploded view of an embodiment of a vaporizer in accordance with the disclosure including a battery segment and a cartomizer positioned for connection with the battery segment, where portions of the exterior surfaces removed to illustrate internal components of the vaporizer;
FIG. 2 illustrates a top view of an embodiment of a vaporizer battery portion;
FIG. 3 illustrates a perspective view of an embodiment of a vaporizer in accordance with the disclosure;
FIG. 4 illustrates a perspective view of an embodiment of a vaporizer in accordance with the disclosure;
FIG. 5 illustrates an exploded perspective view of an embodiment of a vaporizer in accordance with the disclosure; and
FIG. 6 illustrates an embodiment of a cartomizer portion which may be utilized with embodiments of a vaporizer in accordance with the disclosure.

### DETAILED DESCRIPTION

The following detailed description and the appended drawings describe and illustrate some embodiments of the disclosure for the purpose of enabling one of ordinary skill in the relevant art to make and use these embodiments. As such, the detailed description and illustration of these embodiments are purely illustrative in nature and are in no way intended to limit the scope of the disclosure in any manner. It should also be understood that the drawings are not necessarily to scale and in certain instances details may have been omitted, which are not necessary for an understanding of the embodiments, such as details of fabrication and assembly. In the accompanying drawings, like numerals represent like components.

In one embodiment of the disclosure In one embodiment of the disclosure, a vaporizer may include a cartomizer and a battery segment. The cartomizer have may have a first cartomizer end and a second cartomizer end. The cartomizer may include an outer cartomizer shell, an internal cavity defined by a portion of the outer cartomizer shell, a heating chamber provided within the outer cartomizer shell and adjacent to the internal cavity, a heating element provided within the heating chamber, a fluid container conformingly dimensioned with the internal cavity, the fluid container insertable and removable from the internal cavity, a mouthpiece provided at or proximate to the second cartomizer end, an airflow channel in fluid communication with the heating chamber and the mouthpiece, cartomizer electrical contacts provided on the outer cartomizer shell proximate the first cartomizer end, and cartomizer electrical circuitry operable to direct an electric current between the cartomizer electrical contacts and the heating element. The battery segment may include an outer battery shell having a first battery end and a second battery end, the cartomizer connectable with the battery segment proximate the first cartomizer end and the first battery end, a battery housed within the outer battery shell, battery electrical contacts provided proximate the first battery end and positioned to contact the cartomizer electrical contacts when the cartomizer is connected to the battery segment, and battery electrical circuitry housed within the battery segment and operable to direct an electrical current between the battery, the battery electrical contacts, the cartomizer electrical contacts, and the heating element. The battery segment further includes a printed circuit board connected to the battery electrical circuitry, wherein the printed circuit board extends through at least a portion of the outer battery shell between the battery and the longitudinal wall of the outer battery shell.

In further embodiments of a vaporizer, a portion of the airflow channel is positioned between the outer cartomizer shell and the fluid container. The cartomizer may further include a piercing element proximate the heating chamber and dimensioned to puncture a base end of the fluid container when the fluid container is received in the internal cavity. A self-healing gasket may be provided at or proximate to the base end of the fluid container, the self-healing gasket thereby sealing the base end of the liquid container after the piercing element is removed. The piercing element may be a hollow needle capable of allowing the vaporizable fluid to travel from the fluid container to the heating chamber through the hollow needle. The cartomizer may further include an adjustable air valve provided to allow air to be drawn into the airflow channel. The vaporizer may further include at least one magnet connected to the battery segment proximate the first end of the battery segment, the at least one magnet operable to secure the cartomizer to the battery segment. The vaporizer may further include at least one magnet connected to the cartomizer proximate the first cartomizer end capable of securing the battery segment to the cartomizer. The battery segment may include at least one charging port capable of charging the battery. The at least one charging port may be arranged on the first end of the battery segment. The at least one charging port may include connectors for coupling to complimentary connectors of a charger. The vaporizer may further include at least one illuminable indicator electrically connected to the printed circuit board. The battery segment may be dimensioned to receive the cartomizer proximate the first battery end. The battery segment and the cartomizer may have substantially cylindrical cross-sections.

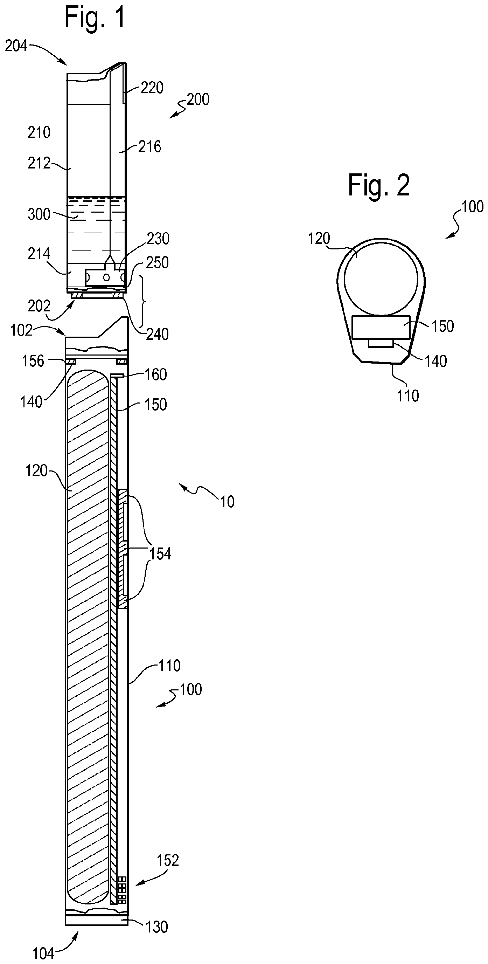

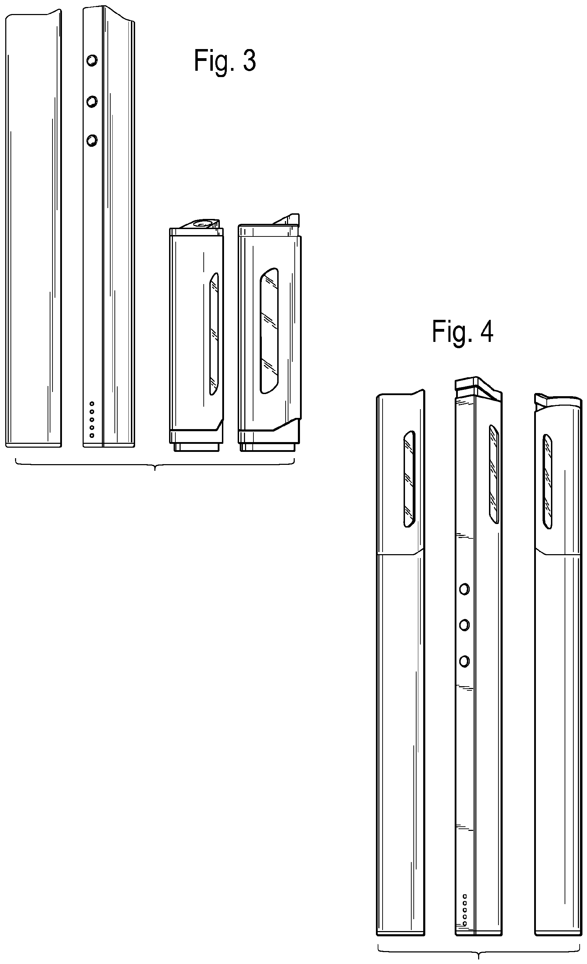

With reference to FIGS. 1-2, an embodiment of a vaporizer 10 may include a battery segment 100, having a cartomizer-side or first end 102 and a charger-side or second end 104, in addition to a cartomizer 200, having a battery-side or first end 202 and a mouthpiece-side or second end 204. Cartomizer 200 may be operable to hold a vaporizable fluid 300. Battery segment 100 may be connectable with the cartomizer 200 through, for example, insertion of the battery-side end 202 of the cartomizer segment into a chamber proximate to the cartomizer-side end 102 of the battery segment 100. While battery segment 100 is illustrated as a substantially elongate member, other dimensions and shapes are contemplated within the disclosure. For instance, and as best illustrated in FIGS. 3 and 4, the battery segment 100 and the cartomizer 200 may be conformingly shaped into a substantially cylindrical or tubular cross-section, with the cartomizer dimensioned to secured on or within a chamber of battery segment 100 proximate to the battery-side end 102. This conformed fit between battery segment 100 and cartomizer 200 may operate to ensure proper fitting and coupling of cartomizer 200 into a chamber of battery segment 100. Other cross-section shapes, including for instance a substantially circular cross section for either battery segment 100 or cartomizer 200, are also contemplated within the disclosure. It is further contemplated that either battery segment 100 or cartomizer 200 may be re-dimensioned so that they are not substantially elongate, as shown in the illustrated embodiments.

Battery segment 100 may include an outer shell 110 for protecting the various components housed internally within at least a portion of shell 110. One component which may be housed within shell 110 is a battery 120, which in some embodiments is rechargeable. In embodiments where battery 120 is rechargeable, a charging port 130 may be provided. In the embodiment illustrated in FIG. 1, charging port 130 is provided proximate to charger-side 104. Charging port may be charged either through direct contact with an electrical source or, alternatively, through inductive charging. In one embodiment, charging port 130 may have connectors, such as threadings or magnets, for directly coupling a charger with charging port 130. For instance, a charger may have male threadings connectable with female threadings provided as part of charging port 130, or charging port 130 may have magnets for securing a charger having magnets of opposing polarities. One such charger with magnet couplings is disclosed in U.S. Application No. 14/201,267. A removable cap could be provided to cover either magnets or threadings so that they are not exposed when not coupled with a charger. The charger may then be connected to a power source, such as through a USB cable or a standard electrical outlet. In another embodiment, charging port 130 may include inductive wireless technology which allows the battery 120 to be charged by placing charging port 130 proximate to an inductive charging pad used for wireless charging. In one embodiment, charging port 130 is dual-functioning in that battery 120 may be charged by either coupling a charger to charging port 130 or placing battery segment 100 near an inductive charging pad, for instance setting battery segment 100 on top of an inductive charging pad.

Battery segment 100 may include more than one charging port 130, where for instance a first charging port and a second charging port are concurrently provided. The first charging port may have the ability to charge battery 120 through a first charging method while the second charging port may have the ability to charge battery 120 through a second charging method. Either the first or second charging method may be direct charging facilitated by directly coupling the charging port with a charger, and the other charging method may be indirect or inductive charging facilitated by placing the associated charging port proximate to an inductive charging pad. In one example embodiment, a first charging port may be provided proximate to charger-side 104 and may be operable to inductively charge battery 120, while a second charging port may be provided proximate to battery-side 102 and may be operable to directly couple with a charger, which may be receivable within the same chamber which cartomizer 200 is insertable into. As a result, various embodiments of vaporizer 10 contemplate battery 120 being rechargeable either through direct coupling of a charger to charging port 130, through inductive charging from placing an inductive charging source proximate to charging port 130.

One or more magnets 140 may be provided in order to secure or couple battery segment 100 with cartomizer 200 as cartomizer 200 is positioned proximate to magnets 140. Magnets 140 may secure the cartomizer 200 through a magnetic attraction with either magnets 240 included with cartomizer 200 or with metal pieces or strips in lieu of active magnets 240. Alternatively, magnets 240 may be provided proximate to battery side 202 of cartomizer 200, and magnets 240 may engage one or more metal strips or pieces 140 in order to establish a magnetic coupling between cartomizer 200 and battery segment 100. Other connectors are contemplated within the disclosure in order to couple battery segment 100 and cartomizer 200 including, for instance, threaded connectors or any other known or to be discovered connector. A snap-fit or friction-fit connection between cartomizer 200 and battery segment 100 is also contemplated within the disclosure.

An electronic control system may be provided in order to operate various functions of vaporizer 10. The electronic control system includes a printed circuit board (PCB) 150, which is substantially elongate and run through at least a portion of the elongate shell 110. In connection with PCB 150 may be one or more indicators 152 as well as one or more control buttons or switches 154. A series of indicators 152, such as the sequentially aligned five (5) indicators shown in the illustrated embodiment of FIG. 1 and FIG. 3, may be operable to indicate a charging or power status of battery segment 100. The indicators may be illuminable, for instance through one or more light emitting diodes (LEDs) included in each indicator 152. Accordingly, each indicator 152 may be composed of one of a series of sequentially drilled holes in a portion of shell 110, with an LED positioned behind the sequentially drilled holes. Buttons 154 may be capacitive touch buttons, manipulable upon pressure or simple contact by a user, or alternatively may be any known or to be developed buttons operable to control the various functions of vaporizer 10 through the electronic control system. A PCB connection 156 may also be provided as part of the electronic control system. Connection 156 may operate to transmit electrical signals to a coupled cartomizer 200 so that electronically controlled functions of cartomizer 200 may be operated through battery segment 100, including through manipulation of buttons 154. Connection 156 may be provided at the base of the chamber proximate to cartomizer-side 102 of battery segment 100. An air switch controller 160 may also be provided and controlled by and through PCB 150.

Cartomizer 200 may include an outer shell 210, the interior of which may include a fluid chamber 212 for holding vaporizable fluid 300. A mouthpiece 220 may be provided on cartomizer 200 at or proximate to mouthpiece-end 204. In some embodiments mouthpiece 220 is releasably attachable to cartomizer 200 in order to expose fluid chamber 212 so that user may add additional fluid 300. Where mouthpiece 220 is removable, mouthpiece 220 may be attached to cartomizer shell 210 by any known or to be developed connector, including threadings or push-in friction fitting. Cartomizer 200 may be divided into a fluid chamber 212 and a heating chamber 214, with the chambers separated by a wall or other barrier. In some embodiments, the heating chamber 214 may be provided proximate to battery-side end 202, while fluid chamber 212 may be provided proximate to mouthpiece-side end 204. A heating or vaporizing unit 230 may be provided within heating chamber 214, where the vaporizing unit is operable to receive fluid 300 from fluid chamber 212 and to heat fluid 300 to a vaporization temperature. Once a vaporization temperature is obtained, fluid 300 transitions to a vapor and may pass through an airflow channel 216 to mouthpiece 220. Airflow channel 216 in one embodiment may be a passageway running between fluid chamber 212 and outer shell 210. In another embodiment, airflow channel 216 may be a composed of a tube or other similar structure extending between heating chamber 214 and mouthpiece 220, through fluid chamber 212. An cartomizer electronic control system may also be provided as part of cartomizer 200, where the cartomizer electronic control system may include a Cartomizer PCB 150. Magnets 240 may also be provided within heating chamber 214.

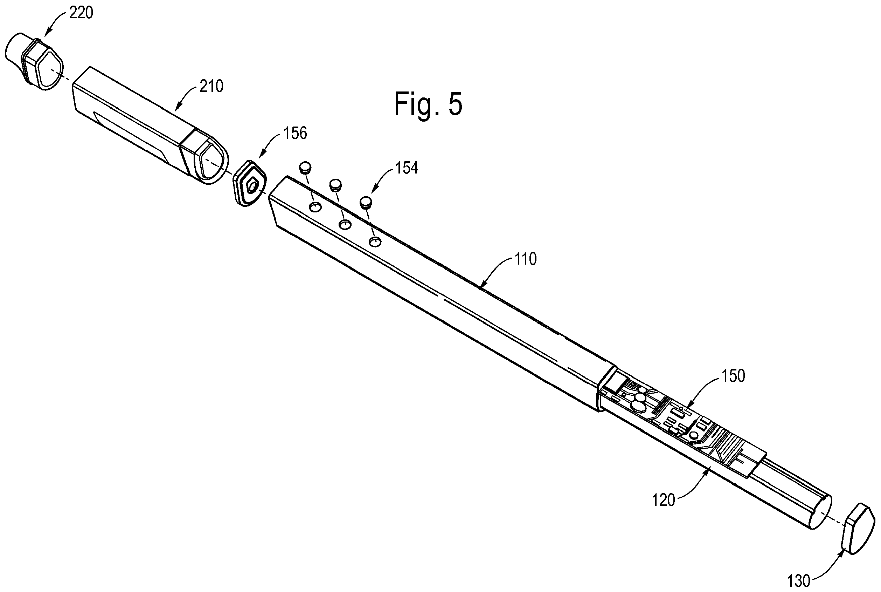

FIGS. 3 and 4 illustrate additional embodiments of a vaporizer in accordance with the disclosure. FIG. 5 illustrates an exploded view of a deconstructed vaporizer, in accordance with the disclosure.

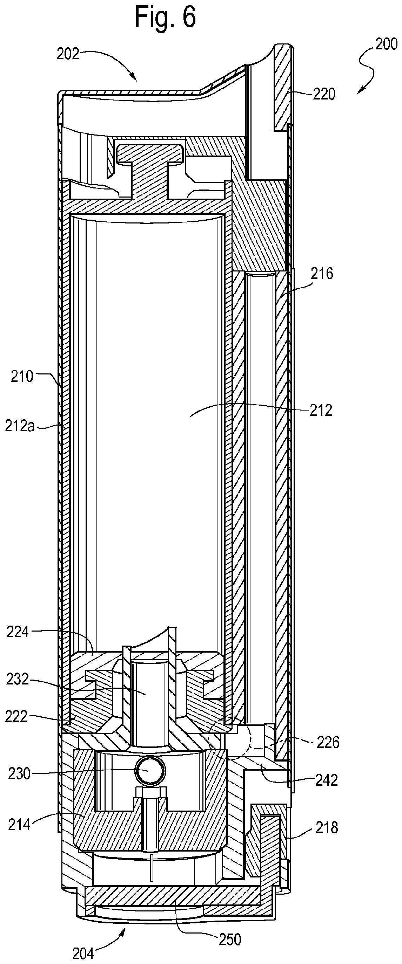

FIG. 6 illustrates an embodiment of cartomizer 200 which may be connected with embodiments of battery segment 100 as part of embodiments of vaporizer 10, in accordance with the disclosure. Cartomizer 200 may include an outer shell 210 having a first end 202 and a mouthpiece end 204. The interior of shell 210 may be dimensioned to receive a removable fluid chamber or capsule 212 having capsule walls 212a. A removable mouthpiece 220 may be provided on cartomizer 200 at or proximate to mouthpiece-end 204. Proximate to first end 202, a heater housing 214 may be provided for holding a heating element 230, which in one embodiment may be a heating coil whose temperature may be elevated to a vaporization temperature upon passing a current through the wound coil. A coupling component 242 may be provided to facilitate connection with battery segment 100 as well as for securing heater housing 214.

Capsule 212 may further include a capsule base 222 and a self-healing rubber gasket 224. As capsule 212 is inserted, an end portion of capsule 212 may be pierced by a puncturing needle 232. In some embodiments, such as the illustrated embodiment, needle 232 may puncture a center area of gasket 224, while in other embodiments needle 232 may puncture a bottom portion of capsule wall 212a or, alternatively, the capsule base 222. In embodiments where needle 232 punctures self-healing rubber gasket 224, the self-healing nature of gasket 224 may allow removal of capsule 212 without leaking, thereby permitting multiple insertion and removals without unintended leaking. Once punctured, vaporizable liquid may travel from capsule 212 through needle 232 to heating element 230 where it may be vaporized to a vaporization temperature and converted from a liquid to an inhalable gas.

An air side channel 216 may be provided for fluidly connecting mouthpiece 220 and heating element 230. In one embodiment, air channel 216 extends longitudinally along a portion of the length of outer shell 210 substantially parallel to an inserted cartridge 212. Environmental air from immediately outside vaporizer 10, may be drawn into an airflow channel through opening 218, which may be an adjustable air valve. By including an adjustable valve at opening 218, users of vaporizer 10 may regulate air flow as may be desirable. A greater or full open opening 218 may produce significantly larger clouds of vapor, while a smaller or partially closed opening 218 may produce smaller but more concentrated vapor. The air channel may continue to be defined along an internal area of cartomizer 200 proximate to first end 202, which for instance may be a portion of coupling component 242, upwards towards heating element 230, and through a gap 226 leading to air side channel 216 and terminating at mouthpiece 220. A PCB 250 may be further provided in order to control various components of cartomizer 200, for instance current control to heating element 230 or operation of valve 218. An electrical current may be provided from battery in battery segment 100 to cartomizer 200 through electrical contacts provided proximate to first end 202.

The descriptions set forth above are meant to be illustrative and not limiting. Various modifications of the invention, in addition to those described herein, will be apparent to those skilled in the art from the foregoing description. Such modifications are also intended to fall within the scope of the concepts described herein.

The foregoing description of possible implementations consistent with the present disclosure does not represent a comprehensive list of all such implementations or all variations of the implementations described. The description of some implementation should not be construed as an intent to exclude other implementations. For example, artisans will understand how to implement the invention in many other ways, using equivalents and alternatives that do not depart from the scope of the invention, which is defined by the appended claims.

### LIST OF REFERENCES

- 10: Vaporizer
- 100: Battery Segment
- 102: First End
- 104: Second End
- 110: Outer Shell
- 120: Battery
- 130: Charging Port
- 140: Magnets
- 150: Printed Circuit Board
- 152: Indicators
- 154: Control Buttons
- 156: PCB connection
- 160: Air Switch Controller
- 200: Cartomizer
- 202: First End
- 204: Second End
- 210: Outer Shell
- 212: Fluid Chamber
- 212a: Capsule Walls
- 214: Heating Chamber
- 216: Airflow Channel
- 218: Opening
- 220: Mouthpiece
- 222: Capsule Base
- 224: Rubber Gasket
- 226: Gap
- 230: Heating Unit
- 232: Needle
- 242: Coupling Component
- 240: Magnets
- 250: Printed Circuit Board
- 300: Vaporizable Fluid

## Claims

1. A vaporizer comprising:
a cartomizer (200) having a first cartomizer end and a second cartomizer end, the cartomizer including;
an outer cartomizer shell (110, 210),
an internal cavity defined by a portion of the outer cartomizer shell,
a heating chamber (214) provided within the outer cartomizer shell and adjacent to the internal cavity,
a heating element (230) provided within the heating chamber,
a fluid container (212) conformingly dimensioned with the internal cavity, the fluid container insertable and removable from the internal cavity,
a mouthpiece (220) provided at or proximate to the second cartomizer end,
an airflow channel (216) in fluid communication with the heating chamber and the mouthpiece,
cartomizer electrical contacts provided on the outer cartomizer shell proximate the first cartomizer end, and
cartomizer electrical circuitry operable to direct an electric current between the cartomizer electrical contacts and the heating element; and
a battery segment (100) including:
an outer battery shell having a first battery end, a second battery end, a longitudinal axis extending between the first battery end and the second battery end, and a longitudinal wall extending along the longitudinal axis, the cartomizer connectable with the battery segment proximate the first cartomizer end and the first battery end,
a battery (120) housed within the outer battery shell,
battery electrical contacts provided proximate the first battery end and positioned to contact the cartomizer electrical contacts when the cartomizer is connected to the battery segment and
battery electrical circuitry housed within the battery segment and operable to direct an electrical current between the battery, the battery electrical contacts, the cartomizer electrical contacts, and the heating element, **characterized in that** an elongated printed circuit board (150) is housed in the battery segment and electrically connected to the battery electrical circuitry, wherein the printed circuit board extends through at least a portion of the outer battery shell between the battery and the longitudinal wall of the outer battery shell.

2. The vaporizer of claim 1, wherein a portion of the airflow channel (216) is positioned between the outer cartomizer shell and the fluid container.

3. The vaporizer of claim 1, wherein the cartomizer further includes a piercing element proximate the heating chamber and dimensioned to puncture a base end of the fluid container when the fluid container is received in the internal cavity.

4. The vaporizer of claim 3, wherein a self-healing gasket (224) is provided at or proximate to the base end of the fluid container, the self-healing gasket thereby sealing the base end of the liquid container after the piercing element is removed.

5. The vaporizer of claim 3, wherein the piercing element is a hollow needle (232) capable of allowing the vaporizable fluid to travel from the fluid container to the heating chamber through the hollow needle.

6. The vaporizer of claim 1, further comprising at least one magnet (140) connected to the battery segment proximate the first end of the battery segment, the at least one magnet operable to secure the cartomizer to the battery segment.

7. The vaporizer of claim 1, further comprising at least one magnet (240) connected to the cartomizer proximate the first cartomizer end capable of securing the battery segment to the cartomizer.

8. The vaporizer of claim 1, wherein the battery segment includes at least one charging port (130) capable of charging the battery.

9. The vaporizer of claim 8, wherein the at least one charging port (130) is arranged on the first end of the battery segment.

10. The vaporizer of claim 8, wherein the at least one charging port (130) includes connectors for coupling to complimentary connectors of a charger.

11. The vaporizer of claim 1, further comprising at least one illuminable indicator (152) electrically connected to the printed circuit board.

12. The vaporizer of claim 1, wherein the battery segment (100) is dimensioned to receive the cartomizer proximate the first battery end.

13. The vaporizer of claim 12, wherein the battery segment and the cartomizer have substantially cylindrical cross-sections.

## Patentansprüche

1. Verdampfer, umfassend
einen Cartomizer (200) mit einem ersten Cartomizerende und einem zweiten Cartomizerende, wobei der Cartomizer umfasst;
eine äußere Cartomizerhülle (110, 210),
einen inneren Hohlraum, der durch einen Teil der äußeren Cartomizerhülle definiert ist
eine Heizkammer (214), die innerhalb der äußeren Cartomizerhülle und neben dem inneren Hohlraum vorgesehen ist,
ein Heizelement (230), das innerhalb der Heizkammer vorgesehen ist,
einen Fluidbehälter (212), der an den inneren Hohlraum angepasst dimensioniert ist, wobei der Fluidbehälter in den inneren Hohlraum einsetzbar und aus diesem herausnehmbar ist,
ein Mundstück (220), das an dem oder in der Nähe des zweiten Cartomizerendes vorgesehen ist,
einen Luftstromkanal (216), der in Fluidverbindung mit der Heizkammer und dem Mundstück steht,
elektrische Cartomizerkontakte, die an der äußeren Cartomizerhülle in der Nähe des ersten Cartomizerendes vorgesehen sind, und
eine elektrische Schaltung für den Cartomizer, die betreibbar ist, um einen elektrischen Strom zwischen den elektrischen Cartomizerkontakten und dem Heizelement zu leiten; und
ein Batteriesegment (100), umfassend:
eine äußere Batteriehülle mit einem ersten Batterieende, einem zweiten Batterieende, einer Längsachse, die sich zwischen dem ersten Batterieende und dem zweiten Batterieende erstreckt, und einer Längswand, die sich entlang der Längsachse erstreckt, wobei der Cartomizer mit dem Batteriesegment in der Nähe des ersten Cartomizerendes und des ersten Batterieendes verbindbar ist,
eine Batterie (120), die innerhalb der äußeren Batteriehülle untergebracht ist,
elektrische Batteriekontakte, die in der Nähe des ersten Batterieendes vorgesehen und so positioniert sind, dass sie die elektrischen Cartomizerkontakte berühren, wenn der Cartomizer mit dem Batteriesegment verbunden ist, und
eine elektrische Batterieschaltung, die innerhalb des Batteriesegments untergebracht und dazu betreibbar ist, einen elektrischen Strom zwischen der Batterie, den elektrischen Batteriekontakten, den elektrischen Cartomizerkontakten und dem Heizelement zu leiten, **dadurch gekennzeichnet, dass**
eine längliche gedruckte Leiterplatte (150), in dem Batteriesegment untergebracht und elektrisch mit der elektrischen Schaltung der Batterie verbunden ist, wobei sich die gedruckte Leiterplatte durch mindestens einen Teil der äußeren Batteriehülle zwischen der Batterie und der Längswand der äußeren Batteriehülle hindurch erstreckt.

2. Verdampfer nach Anspruch 1, wobei ein Teil des Luftstromkanals (216) zwischen der äußeren Cartomizerhüller und dem Fluidbehälter angeordnet ist.

3. Verdampfer nach Anspruch 1, wobei der Cartomizer ferner ein Durchstechelement in der Nähe der Heizkammer aufweist, das so dimensioniert ist, dass es ein Basisende des Fluidbehälters durchsticht, wenn der Fluidbehälter in dem inneren Hohlraum aufgenommen ist.

4. Verdampfer nach Anspruch 3, wobei eine selbstheilende Dichtung (224) and dem oder in der Nähe des Basisendes des Fluidbehälters vorgesehen ist, wodurch die selbstheilende Dichtung das Basisende des Fluidbehälters abdichtet, nachdem das Durchstechelement entfernt wurde.

5. Verdampfer nach Anspruch 3, wobei das Durchstoßelement eine Hohlnadel (232) ist, die es dem verdampfbaren Fluid ermöglicht, durch die Hohlnadel von dem Fluidbehälter zu der Heizkammer zu gelangen.

6. Verdampfer nach Anspruch 1, ferner umfassend mindestens einen Magneten (140), der mit dem Batteriesegment in der Nähe des ersten Endes des Batteriesegments verbunden ist, wobei der mindestens eine Magnet betreibbar ist, um den Cartomizer an dem Batteriesegment zu befestigen.

7. Verdampfer nach Anspruch 1, ferner umfassend mindestens einen Magneten (240), der mit dem Cartomizer in der Nähe des ersten Endes des Cartomizers verbunden ist und der dazu in der Lage ist, das Batteriesegment an dem Cartomizer zu befestigen.

8. Verdampfer nach Anspruch 1, wobei das Batteriesegment mindestens einen Ladeanschluss (130) aufweist, der zum Laden der Batterie geeignet ist.

9. Verdampfer nach Anspruch 8, wobei der mindestens eine Ladeanschluss (130) an dem ersten Ende des Batteriesegments angeordnet ist.

10. Verdampfer nach Anspruch 8, wobei der mindestens eine Ladeanschluss (130) Anschlüsse zur Kopplung mit komplementären Anschlüssen eines Ladegeräts umfasst.

11. Verdampfer nach Anspruch 1, ferner umfassend mindestens ein aufhellbares Anzeigeelement (152), das elektrisch mit der Leiterplatte verbunden ist.

12. Verdampfer nach Anspruch 1, wobei das Batteriesegment (100) dazu dimensioniert ist, den Cartomizer in der Nähe des ersten Batterieendes aufzunehmen.

13. Verdampfer nach Anspruch 12, wobei das Batteriesegment und der Cartomizer im Wesentlichen zylindrische Querschnitte aufweisen.

## Revendications

1. Vaporisateur comprenant :
un cartomiseur (200) ayant une première extrémité de cartomiseur et une seconde extrémité de cartomiseur, le cartomiseur comprenant ;
une coque externe de cartomiseur (110, 210),
une cavité interne définie par une partie de la coque externe de cartomiseur,
une chambre de chauffage (214) prévue à l'intérieur de la coque externe de cartomiseur et adjacente à la cavité interne,
un élément chauffant (230) disposé à l'intérieur de la chambre de chauffage,
un conteneur de fluide (212) dimensionné conformément à la cavité interne, le conteneur de fluide pouvant être inséré et retiré de la cavité interne,
un embout buccal (220) prévu au niveau ou à proximité de la seconde extrémité de cartomiseur,
un canal d'écoulement d'air (216) en communication fluidique avec la chambre de chauffage et l'embout buccal,
des contacts électriques de cartomiseur disposés sur la coque externe de cartomiseur à proximité de la première extrémité de cartomiseur, et
un circuit électrique de cartomiseur pouvant fonctionner pour diriger un courant électrique entre les contacts électriques de cartomiseur et l'élément chauffant ; et
un segment de batterie (100) comprenant :
une enveloppe externe de batterie ayant une première extrémité de batterie, une seconde extrémité de batterie, un axe longitudinal s'étendant entre la première extrémité de batterie et la seconde extrémité de batterie, et une paroi longitudinale s'étendant le long de l'axe longitudinal, le cartomiseur pouvant être connecté au segment de batterie à proximité de la première extrémité de cartomiseur et de la première extrémité de batterie, une batterie (120) logée à l'intérieur de l'enveloppe externe de batterie,
des contacts électriques de batterie disposés à proximité de la première extrémité de batterie et positionnés pour entrer en contact avec les contacts électriques de cartomiseur lorsque le cartomiseur est connecté au segment de batterie et
un circuit électrique de batterie logé dans le segment de batterie et pouvant fonctionner pour diriger un courant électrique entre la batterie, les contacts électriques de la batterie, les contacts électriques de cartomiseur et l'élément chauffant,
**caractérisé en ce qu'**une carte de circuit imprimé allongée (150) est logée dans le segment de batterie et connectée électriquement au circuit électrique de batterie, dans laquelle la carte de circuit imprimé s'étend à travers au moins une partie de l'enveloppe externe de batterie entre la batterie et la paroi longitudinale de l'enveloppe externe de batterie.

2. Vaporisateur selon la revendication 1, dans lequel une partie du canal d'écoulement d'air (216) est positionnée entre la coque externe de cartomiseur et le conteneur de fluide.

3. Vaporisateur selon la revendication 1, dans lequel le cartomiseur comprend en outre un élément de perçage à proximité de la chambre de chauffage et dimensionné pour percer une extrémité de base du conteneur de fluide lorsque le conteneur de fluide est reçu dans la cavité interne.

4. Vaporisateur selon la revendication 3, dans lequel un joint autoréparable (224) est disposé au niveau ou à proximité de l'extrémité de base du conteneur de fluide, le joint autoréparable scellant ainsi l'extrémité de base du récipient de liquide après le retrait de l'élément de perçage.

5. Vaporisateur selon la revendication 3, dans lequel l'élément de perçage est une aiguille creuse (232) apte à permettre au fluide vaporisable de se déplacer du conteneur de fluide vers la chambre de chauffage à travers l'aiguille creuse.

6. Vaporisateur selon la revendication 1, comprenant en outre au moins un aimant (140) connecté au segment de batterie à proximité de la première extrémité du segment de batterie, l'au moins un aimant pouvant fonctionner pour fixer le cartomiseur au segment de batterie.

7. Vaporisateur selon la revendication 1, comprenant en outre au moins un aimant (240) connecté au cartomiseur à proximité de la première extrémité de cartomiseur apte à fixer le segment de batterie au cartomiseur.

8. Vaporisateur selon la revendication 1, dans lequel le segment de batterie comprend au moins un port de charge (130) apte à charger la batterie.

9. Vaporisateur selon la revendication 8, dans lequel l'au moins un port de charge (130) est disposé sur la première extrémité du segment de batterie.

10. Vaporisateur selon la revendication 8, dans lequel l'au moins un port de charge (130) comprend des connecteurs pour le couplage à des connecteurs complémentaires d'un chargeur.

11. Vaporisateur selon la revendication 1, comprenant en outre au moins un indicateur lumineux (152) connecté électriquement à la carte de circuit imprimé.

12. Vaporisateur selon la revendication 1, dans lequel le segment de batterie (100) est dimensionné pour recevoir le cartomiseur à proximité de la première extrémité de batterie.

13. Vaporisateur selon la revendication 12, dans lequel le segment de batterie et le cartomiseur ont des sections transversales sensiblement cylindriques.
